# EUROPÄISCHE PATENTSCHRIFT

(11) **EP 1 992 405 B1**
(45) Veröffentlichungstag und Bekanntmachung des Hinweises auf die Patenterteilung: **20.05.2009**
(21) Anmeldenummer: 08103822.6
(22) Anmeldetag: 05.05.2008
(51) Int. Cl.: B01F 7/02, B01F 7/06, B01F 15/00, B01F 3/12, A01C 3/02, C12M 1/02

(54) **Fördersystem eines Gär- bzw. Faulbehälters**
Conveying system of a fermentation/digestion tank
Système de transport d'un récipient de fermentation ou de pourriture

(30) Priorität: 14.05.2007 DE 102007022902
(43) Veröffentlichungstag der Anmeldung: 19.11.2008
(73) Patentinhaber: Karl Buschmann Maschinenbau GmbH, 46499 Hamminkeln (DE)
(72) Erfinder:
(74) Vertreter: Mederle-Hoffmeister, Stefan

(56) Entgegenhaltungen:
- EP-A- 1 932 902
- CH-A- 129 230
- DE-A1- 2 855 206
- DE-A1- 19 756 485
- DE-U1- 8 714 525
- DE-U1- 9 404 188
- US-A- 4 836 687

## Beschreibung

Die Erfindung betrifft ein Fördersystem eines Gär- bzw. Faulbehälters, insbesondere für Biogas-Anlagen bzw. biologische Kläranlagen gemäß dem Oberbegriff des Patentanspruchs 1, sowie einen Gär- bzw. Faulbehälter gemäß dem Oberbegriff des Patentanspruchs 10.

Die US 4 836 687 A1 beschreibt eine Rührvorrichtung zum Mischen eines nicht homogenen Abfallmaterials in einem Behälter. Die Rührvorrichtung weist eine angetriebene Welle auf, an deren vorderem Ende einklappbare Schaufeln befestigt sind, so dass die Rührvorrichtung leicht in im Durchmesser kleinere Einlassöffnungen des Behälters eingeführt bzw. daraus entfernt werden kann.

Aus der DE 94 04 188 U1 ist eine Mischeinrichtung für flüssigkeitsgefüllte Behälter bekannt. Diese Mischeinrichtung umfasst unter anderem ein mit einer Führungseinheit fest verbundenes Mischelement, das mittels einer Hebevorrichtung in den Behälter abzusenken und aus diesem zu heben ist. Ferner weist sie eine dem Mischelement und dessen Führungseinheit zugeordnete Führungs- und Halterungsvorrichtung auf, die im Wesentlichen aus einem auf dem Behälterboden angeordneten, im Bereich seiner Enden befestigten Führungsrohr und aus einer am Führungsrohr befestigten Auflage für das Mischelement und/oder dessen Führungseinheit gebildet wird. Am Rand des Behälters ist ein begehbares Podest angeordnet, an dessen vom Rand des Behälters abgewandten Vorderseite das Führungsrohr befestigt ist. Der Abstand zwischen dem Führungsrohr und der Wand des Behälters ist so bemessen, dass das Mischelement die für seine ungestörte Funktion notwendigen saugseitigen radialen Freiräume besitzt.

Eine derartige Fördereinrichtung, bei welcher das Mischelement aus einer Hebevorrichtung in den Behälter abgesenkt wird, ist jedoch konstruktiv relativ aufwendig und dementsprechend personal- und wartungsintensiv.

Weiterhin ist aus der DE 197 56 485 A1 ein Gärbehälter mit einer runden Bodenfläche und einem Füllstutzen sowie einer am Umfang des Gärbehälters angebrachten Fördereinrichtung in Form eines Rührwerks mit einer Antriebsachse bekannt. Das Rührwerk ist in einem unterhalb des Füllstutzens angeordneten Rührrohr angeordnet, und die verlängerte horizontale Projektion der Antriebsachse schneidet den Umfang des Gärbehälters sekantenförmig.

Durch das Rührwerk wird für eine Durchmischung der Gärstoffe im Gärbehälter gesorgt. Das Rührwerk setzt sich aus einem Motor, einer Antriebsachse bzw. einer Antriebswelle und einem Förderelement in Form eines Rührkopfes, welcher gemäß der DE 197 56 485 A1 schrauben- oder propellerförmig ausgebildet sein kann, zusammen. Während sich der Motor außerhalb des Gärbehälters befindet, ist der Rührkopf innerhalb eines Prozessraums des Gärbehälters angeordnet. Die Wirkverbindung zwischen Motor und Rührkopf wird über eine Antriebsachse bzw. Antriebswelle sichergestellt, welche in einer korrespondierenden Durchführung durch die Behälterwand des Gärbehälters hindurch geführt ist. Wegen der hohen Beanspruchung treten gerade bei der Fördereinrichtung häufig Defekte bzw. Verschleißerscheinungen auf, sodass es nötig ist, die Fördereinrichtung aus dem Behälter zu entfernen. Dazu muss der Behälter jedes Mal vollständig entleert werden, was für hohe Betriebskosten und einen unerwünschten Produktionsausfall sorgt.

Aufgabe der vorliegenden Erfindung ist es demnach, ein Fördersystem eines Gär- bzw. Faulbehälters anzugeben, welches eine gute Durchmischung der Faul- bzw, Gärstoffe bei gleichzeitig verringerten Produktionsausfällen sicherstellt. Ferner ist es Aufgabe der vorliegenden Erfindung, einen Gär- bzw. Faulbehälter anzugeben, der bei einer guten Durchmischung der Gärstoffe gleichzeitig einen geringen Wartungsaufwand sicherstellt. Diese Aufgabe wird erfindungsgemäß durch ein Fördersystem eines Gär- bzw. Faulbehälters gemäß Patentanspruch 1, sowie durch einen Gärbehälter gemäß Patentanspruch 10 gelöst.

Weitere Merkmale der Erfindung sind in den Unteransprüchen enthalten.

Hinsichtlich der Terminologie der vorliegenden Anmeldung sei an dieser Stelle angemerkt, dass, wenn in der Folge ein Gärbehälter genannt ist, es sich immer um einen Gär- bzw. Faulbehälter handelt. Entsprechend handelt es sich bei der Nennung von Gärstoffen immer um Gär- bzw. Faulstoffe und bei einem Gärstoffkreislauf immer um einen Gär- bzw. Faulstoffkreislauf.

Die Erfindung wird im Folgenden mit Bezug auf die Zeichnungen anhand von bevorzugten Ausführungsformen beispielhaft beschrieben. Die Zeichnungen zeigen in:
- Fig. 1: eine erste bevorzugte Ausführungsform eines erfindungsgemäßen Fördersystems, eingebaut in einen Gärbehälter, in einer Querschnitts-Darstellung;
- Fig. 2: eine zweite bevorzugte Ausführungsform eines erfindungsgemäßen Fördersystems, eingebaut in einen Gärbehälter, wiederum in einer Querschnittsdarstellung;
- Fig. 3: eine dritte bevorzugte Ausführungsform eines erfindungsgemäßen Fördersystems, wiederum eingebaut in einen Gärbehälter und in Querschnittsdarstellung;
- Fig. 4: eine schematische Darstellung des Fördersystems und eines Teilabschnitts des Gärbehälters der ersten bevorzugten Ausführungsform;
- Fig. 5: eine schematische Darstellung des Fördersystems sowie eines Teilabschnitts des Gärbehälters der zweiten und dritten bevorzugten Ausführungsform in einer Teilexplosionsdarstellung;
- Fig. 6: das Fördersystem und den Teilabschnitt des Gärbehälters gemäß Fig. 5 in zusammengesetztem Zustand;
- Fig. 6a: das Fördersystem gemäß Fig. 6 in einer weiteren möglichen Betriebsstellung, wiederum in einer schematischen Ansicht; und
- Fig. 7: eine weitere mögliche bevorzugte Ausführungsform eines erfindungsgemäßen Fördersystems.

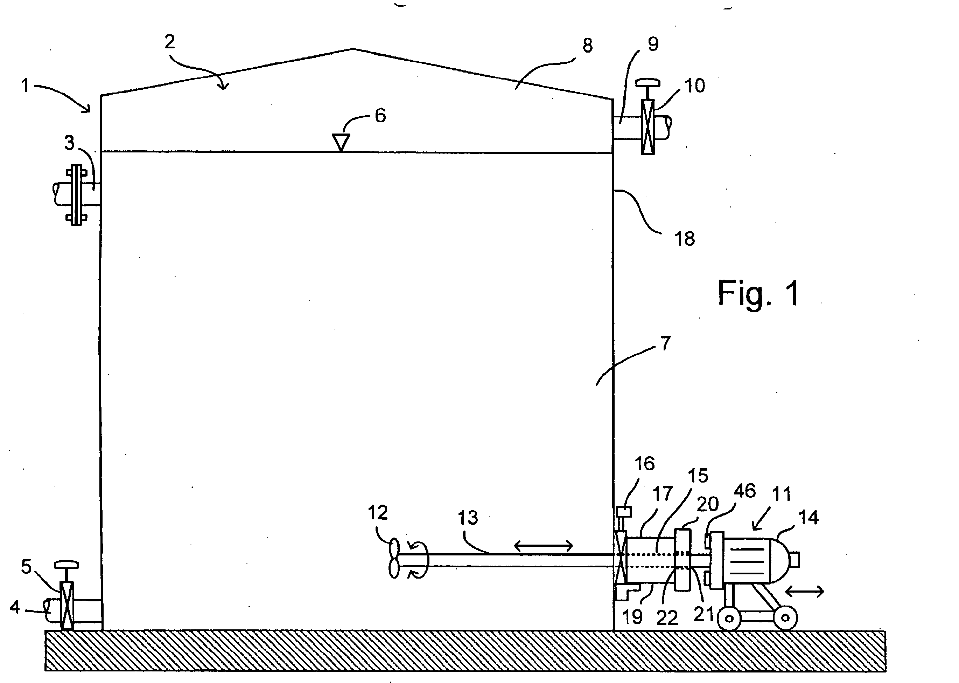

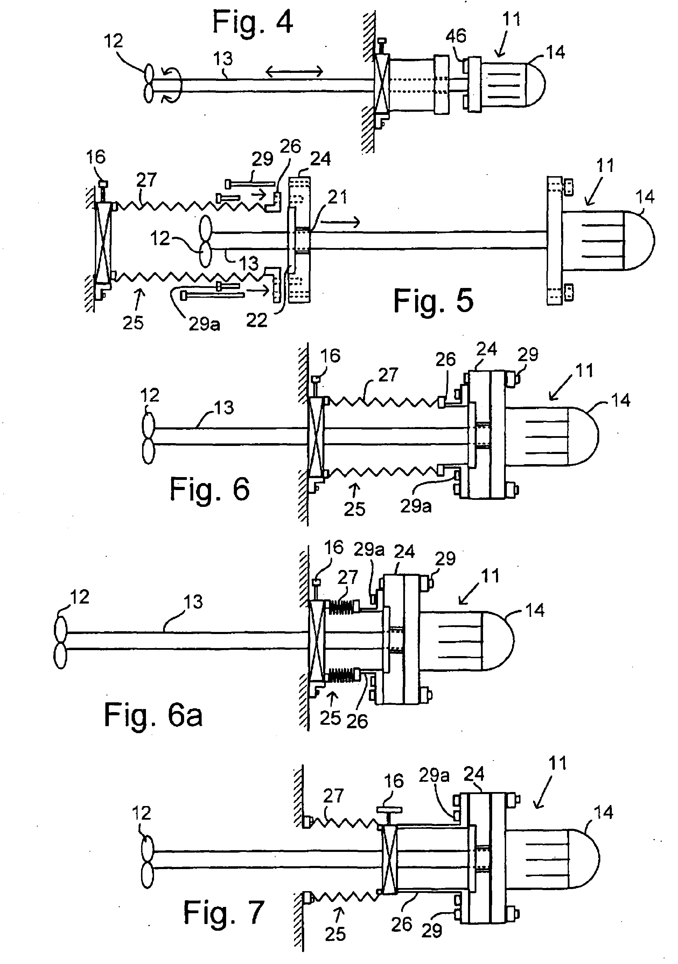

Zur Verdeutlichung ihrer Funktion und ihres Einsatzgebietes seien die erfindungsgemäßen Fördersysteme hauptsächlich in einem in einem Gär- bzw. Faulbehälter 1 eingebauten Zustand (Fig. 1 bis 3) erläutert, während Details anhand der Fig. 4 bis 7 erläutert seien.

Die erste bevorzugte Ausführungsform eines erfindungsgemäßen Fördersystems ist an einen Gär- bzw. Faulbehälter 1 (vgl. Fig. 1) angebaut, welcher einen Prozessraum 2, in welchem die Biogaserzeugung stattfindet, sowie einen Einlass 3, welcher zur Befüllung, und einen Auslass 4, welcher zur Entleerung des Gärbehälters 1 dient, umfasst. Am Auslass 4 ist eine Absperrvorrichtung 5 in Form eines Schiebers angeordnet, welcher eine unerwünschte Entleerung des Behälters verhindert. Alternativ zu einem Schieber kann die Absperrvorrichtung 5 auch in Form eines Ventils, beispielsweise in Form eines Kugelventils oder eines Klappenventils vorliegen. Der Schieber ist manuell betätigbar, wobei auch eine automatisierte Betätigung auf elektrischem, pneumatischem oder hydraulischem Wege denkbar ist.

Der Prozessraum 2 ist bis zu einem Betriebsfüllstand 6 mit Gär- bzw. Faulstoffen 7 gefüllt, während oberhalb des Betriebsfüllstands 6 das anfallende Gas in einem Gasraum 8 gesammelt und über einen Gasauslass 9 seiner Verwendung zugeführt wird. Der Gasauslass 9 weist eine gasdichte Absperrvorrichtung 10 auf.

Bei dem Behälter 1 kann es sich beispielsweise um einen Blechstau-, einen Kunststoff- oder einen gemauerten Behälter handeln, welcher ggf. eine entsprechende gasdichte Auskleidung besitzt. Im Bereich gemauerter Behälter kommen insbesondere Behälter aus Beton, welche entsprechende Kernbohrungen als Öffnungen aufweisen, in Frage.

Das Fördersystem der ersten bevorzugten Ausführungsform, welches in Fig. 1 in den Gärbehälter 1 integriert ist, weist eine Misch- bzw. Förderreinrichtung 11 auf, welche ein Misch- bzw. Förderelement in Form eines Propellers 12, eine Antriebswelle 13 sowie eine Antriebsvorrichtung in Form eines Motors 14 umfasst. Der Propeller 12 steht über die Antriebswelle 13 mit dem Motor 14 kraftschlüssig in Wirkeingriff. Der Kraftübertrag zwischen Motor 14 und Antriebswelle 13 kann beispielsweise über ein Getriebe oder eine anderweitige Antriebsmethode, insbesondere einen Ketten- oder Riemenantrieb erfolgen. An Stelle eines Propellers 12 als Förderelement ist selbstverständlich auch eine Schraube (beispielsweise eine schiffschraubenartig ausgebildete zwei- oder dreiblättrige Schraube), eine Schnecke oder dergleichen Vorrichtung denkbar.

Um eine gute Durchmischung der zu Gär- bzw. Faulstoffe 7 im Prozessraum 2 zu gewährleisten, ist die Misch- bzw. Fördereinrichtung 11 positionsveränderlich gegenüber dem Faul- bzw. Gärbehälter 1 angeordnet. In der vorliegenden bevorzugten Ausführungsform ist dabei vorgesehen, einen Teil der Fördervorrichtung 11, nämlich den Propeller 12 und Teile der Antriebswelle im Betriebszustand positionsveränderlich im Prozessraum 2 anzuordnen. In der Ausführungsform gemäß Fig. 1 ist die Positionsveränderlichkeit dadurch realisiert, dass die Misch- bzw. Fördereinrichtung 11 in Richtung der Antriebswellenmittelachse verschieblich zum Gär- bzw. Faulbehälter 1 gelagert bzw. geführt ist, was für eine gute Durchmischung der Gär- bzw. Faulstoffe sorgt. In der vorliegenden bevorzugten Ausführungsform ist die Antriebswelle 13 im Bereich des Motors 14 in einem Schrägwellenlager gelagert, während sie weiter zum Gärbehälter 1 hin lediglich geführt ist (Führungsbuchse 21). Alternativ hierzu ist auch eine (ggf. weitere) Lagerung der Antriebswelle 13 im Bereich des Gärbehälters 1 denkbar.

Das Fördersystem umfasst weiterhin eine Kammer 15, welche teilweise durch eine Kammerwand 17 begrenzt ist. Ein zwischen der Kammer 15 und dem Prozessraum 2 angeordnetes Absperrelement in Form eines Schiebers 16 kann alternativ geöffnet oder geschlossen werden um die Kammer 15 mit dem Prozessraum 2 zu verbinden oder von letzterem flüssigkeitsdicht oder fluiddicht zu trennen. Aufgrund der Verschieblichkeit der Misch- bzw. Fördereinrichtung 11 ist es möglich, die Antriebswelle 13 mit dem daran befestigten Propeller 12 durch den geöffneten Schieber 16 hindurch in den Prozessraum 2 einzuführen und aus diesem in die Kammer 15 zurückzuziehen. Dies ermöglicht nach einem Schließen des Schiebers 16 einen einfachen Austausch des Propellers 12 und/oder weiterer Komponenten der Fördereinrichtung 11. Zum Zurückziehen des Propellers 12 in die Kammer 15 ist die Öffnung in der Behälterwand 18 und auch der lichte Querschnitt des Schiebers 16 derart ausgestaltet, dass der Propeller 12 durch die jeweilige Öffnung hindurchgeführt werden kann. Alternativ zum Schieber 16 sind auch anderweitige Absperrelemente wie beispielsweise ein Kugelventil, ein Klappenventil oder dergleichen denkbar. Die Verschieblichkeit der Fördereinrichtung 11 wird in der vorliegenden Ausführungsform durch Räder unterstützt, welche am Motor über entsprechende Stützelemente angebracht sind und z.B, auf Schienen (nicht dargestellt) verschiebbar geführt sind. Alternativ hierzu sind auch andere Verschiebemechanismen wie Hydraulikvorrichtungen o.ä. denkbar. Um einen zu harten Aufprall des Motors 14 der Fördereinrichtung 11 an einer Abschlussplatte 20 zu verhindern, sind Pufferelemente 46 am Motor 14 angebracht.

Die Kammer 15 ist wie bereits vorstehend beschrieben auf ihrer einen Seite, nämlich der der Behälterwand zugewandten (Stirn-) Seite 18 durch den Schieber 16 begrenzt, während sie in radialer Richtung durch ein röhrenförmiges Element 19, welches die Kammerwand 17 bildet, begrenzt ist. Auf der dem Gärbehälter 1 abgewandten (Stirn-) Seite ist an dem röhrenförmigen Element 19 eine Abschlussplatte 20 angebracht, welche in der Mitte eine Aussparung zur Durchführung der Antriebswelle 13 aufweist. Die Antriebswelle 13 ist in der in die Abschlussplatte 20 integrierten Führungsbuchse 21 geführt, wobei ferner auf der dem Behälter zugewandten Seite eine ebenfalls in die Abschlussplatte 20 integrierte Dichtung (Antriebswellendichtung) in Form eines Simmerings 22 für die nötige Abdichtung sorgt. Alternativ oder zusätzlich zum Simmering 22 kann die Dichtung andere/weitere Dichtelemente wie z. B. eine Labyrinth-Dichtung, einen O-Ring, eine Dichtungsbuchse oder aber andere Dichtungselemente in Form einer Manschette aufweisen.

Die bevorzugte Ausführungsform ist nochmals detailliert in Fig.4 dargestellt.

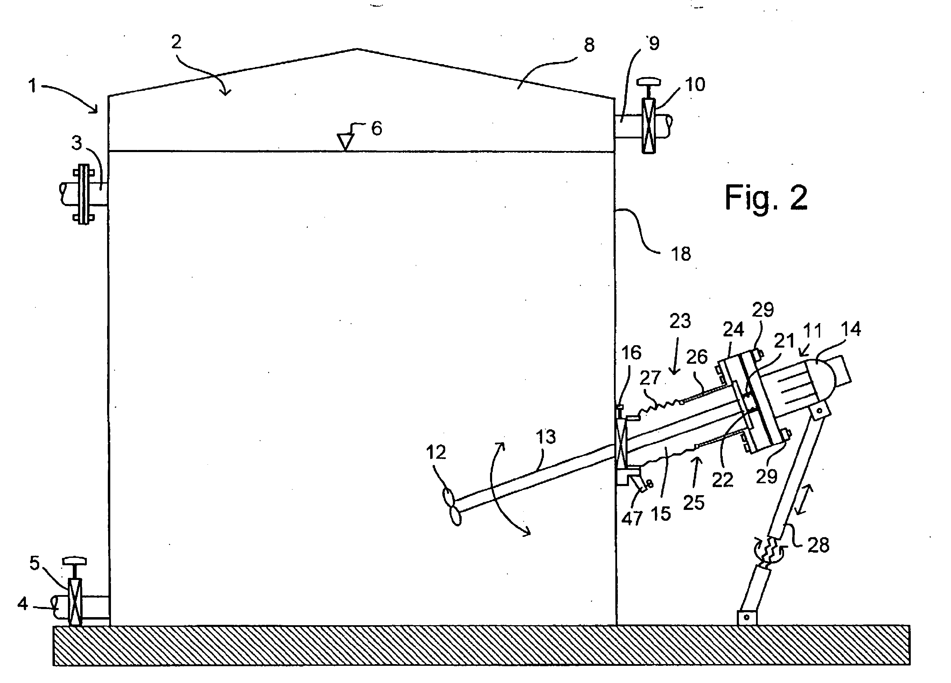

Der Gärbehälter 1, in den die zweite bevorzugte Ausführungsform eines erfindungsgemäßen Fördersystems integriert ist (vgl. Fig. 2), entspricht demjenigen, in den die erste Ausführungsform integriert ist. In der Folge sei deshalb auf die Unterschiede zwischen der ersten und der zweiten bevorzugten Fördersystem-Ausführungsform eingegangen.

Beim Fördersystem gemäß der zweiten bevorzugten Ausführungsform ist neben einer Verschieblichkeit des Propellers 12 entlang der Antriebswellenmittelachse zusätzlich eine Schwenkbarkeit desselben relativ zum Gärbehälter 1 gegeben. Dadurch kann die Durchmischung der Gärstoffe nochmals verbessert werden. Der Schwenkbereich der Misch- bzw. Fördereinrichtung 11 in vertikaler Richtung liegt in einem Schwenkwinkelintervall von 20° bis 30° (von einer Horizontalen nach unten gerichtet). Als Schwenkwinkel ist dabei der Winkel definiert, den die Antriebswellenmittelachse mit einer gedachten horizontalen Linie einschließt.

In der zweiten bevorzugten Ausführungsform wird die Kammer 15 durch eine Befestigungsvorrichtung 23 gebildet, an welcher die Fördereinrichtung 11 befestigt ist und welche selbst wiederum am Gärbehälter 1 befestigt ist. Diese umfasst ein Zwischenelement in Form einer Zwischenplatte 24 sowie ein Anschlusselement 25, welches an seiner dem Gärbehälter 1 abgewandten Seite an der Zwischenplatte 24 befestigt ist und an seiner dem Gärbehälter 1 zugewandten Seite am Gärbehälter 1 selbst bzw. dessen Wand 18 befestigt bzw. befestigbar ist. Die Zwischenplatte 24 weist analog zu der Abschlussplatte 20 eine Führungsbuchse 21 auf, in welcher die Antriebswelle 13 geführt ist. Als Dichtungselement ist ebenfalls analog zur ersten bevorzugten Ausführungsform ein Simmering 22 vorhanden, wobei auf die Alternativen, die im Zusammenhang mit der ersten bevorzugten Ausführungsform erwähnt werde, verwiesen sei. Die Zwischenplatte 24 ist auf ihrer dem Gärbehälter 1 abgewandten Seite mit dem Motor 14 mit Befestigungselementen in Form von z.B. Schrauben 29 (alternativ können Bolzen oder andersartige Befestigungselemente Verwendung finden) verbunden, während sie auf ihrer dem Gärbehälter 1 zugewandten Seiten mit dem Anschlusselement 25 verbunden, vorzugsweise verschraubt ist.

Das Anschlusselement 25 setzt sich aus einem röhren- bzw. schlauchartigen Abschnitt 26, einem aus einem Faltenbalg 27 bestehenden Abschnitt sowie dem Schieber 16 zusammen. Der Faltenbalg 27 ist aus einem geeigneten, gärstoffbeständigen Kunststoff (alternativ aus einem geeigneten Metall) gefertigt und mit dem bereits aus der ersten bevorzugten Ausführungsform bekannten Schieber 16 verbunden. Es sei an dieser Stelle angemerkt, dass an Stelle des Faltenbalgs auch ein anderes flexibles Element, beispielsweise ein schlauchartiges Element, denkbar wäre.

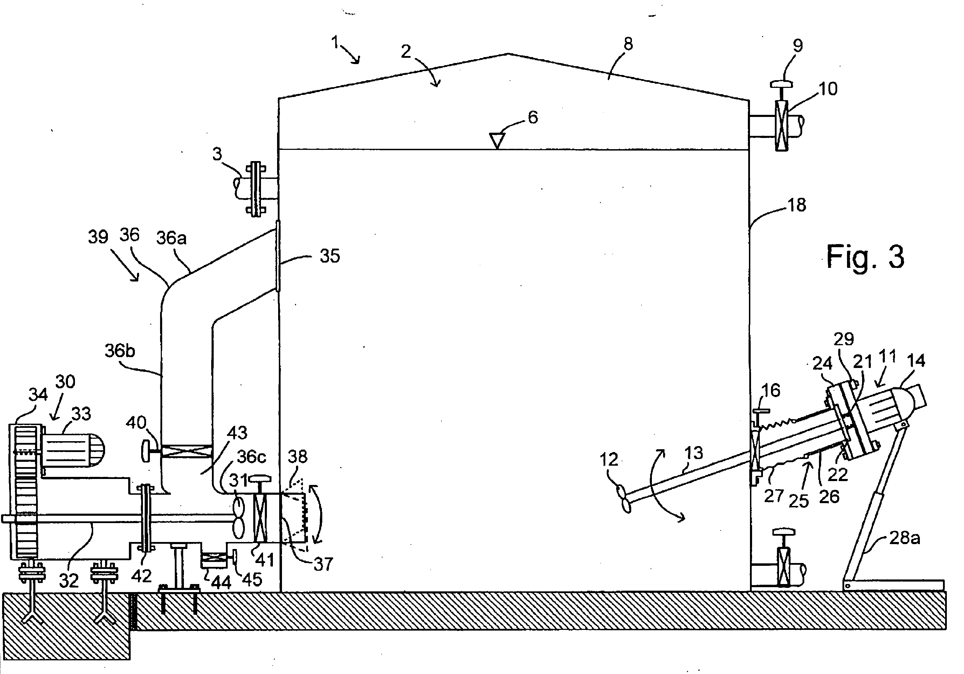

Für eine korrekte Einstellung des Schwenkwinkels gegenüber der Horizontalen weist die zweite bevorzugte Ausführungsform eine verstellbare Stütze für die Fördereinrichtung 11 in Form einer Gewindespindel 28 auf. Dadurch kann der gewünschte Schwenkwinkel in einer einfachen Art und Weise realisiert werden. Alternativ hierzu wären auch eine Hydraulikvorrichtung (vgl. hierzu auch Fig. 3) oder eine andere (verstellbare) Stützvorrichtung denkbar.

In der zweiten bevorzugten Ausführungsform ist neben der Herausnehmbarkeit bzw. Rückziehbarkeit des Förderelements 12 in die Kammer 15, wie bereits vorstehend erwähnt, für eine bessere Durchmischung (aufgrund der Verschieblichkeit und einer zusätzlichen Schwenkbarkeit des Förderelements) gesorgt. Während im Betrieb durch eine Längsverschieblichkeit und Schwenkbarkeit durch den Faltenbalg 27 eind optimale Durchmischung ermöglicht wird, besteht nach einem Lösen der Schrauben 29 die Möglichkeit, die Misch- bzw. Fördereinrichtung 11 von der Befestigungsvorrichtung 23 zu trennen und den Propeller 12 danach in die Kammer 15, welche durch die Zwischenplatte 24, das Anschlusselement 25 und den Schieber 16 begrenzt ist, zurückzuziehen. Nach dem Zurückziehen kann der Schieber 16 flüssigkeitsdicht bzw. in einer weiteren bevorzugten Ausführungsform fluiddicht geschlossen werden. Einem einfachen Austausch des Misch- bzw. Förderelements 12 oder der gesamten Misch- bzw. Fördereinrichtung 11 durch ein Abflanschen der Zwischenplatte 24 (bzw. in der ersten bevorzugten Ausführungsform der Abschlussplatte 20) steht nichts mehr entgegen. Um eine Entleerung der Kammer 15 vor dem Abflanschen zu ermöglichen, weist die Befestigungsvorrichtung einen verschliessbaren Auslass 47 auf, welcher ein Ablassen der in der Kammer befindlichen Gärstoffe ermöglicht. Auch die anderen bevorzugten Ausführungsformen können einen solchen Auslass 47 aufweisen.

In Zusammenhang mit der zweiten bevorzugten Ausführungsform sei auf die Figuren 5 bis 7 verwiesen. Wie aus Fig. 5 hervorgeht, kann sowohl nur die Zwischenplatte 24 mit dem Motor mittels der Schrauben 29 verschraubt sein, während aber auch eine durchgehende Verschraubung von Anschlusselement 25, Zwischenplatte 24 und Motor 14 denkbar ist. Das Anschlusselement kann (ggf. zusätzlich zu den Schrauben 29) mit Befestigungselementen in Form von Schrauben 29a an der Zwischenplatte 24 befestigt sein.

In den Figuren 6 und 6a ist die Misch- bzw. Fördereinrichtung 11 gemäß Fig. 5 in zusammengebautem Zustand sowohl mit gestrecktem (Fig. 6) als auch mit komprimiertem (Fig. 6a) Faltenbalg dargestellt. Aus Fig. 7 letztendlich kann man ersehen, dass die Position des Schiebers auch zwischen dem röhren- bzw. schlauchartigen Abschnitt 26 und dem Faltenbalg 27 denkbar ist.

Es sei an dieser Stelle angemerkt, dass der Grundgedanke der Erfindung nicht nur die Bereitstellung eines Fördersystems, sondern auch die Bereitstellung eines vollständigen Gär- bzw. Faulbehälter mit einem erfindungsgemäßen Fördersystem umfasst.

In einer bevorzugten Ausführungsform eines Gärbehälters 1 (vgl. Fig. 3) weist das Fördersystem nahezu alle Merkmale der aus der Fig. 2 bekannten Ausführungsform auf, mit der Ausnahme, dass anstatt der Gewindespindel 28 ein Hydraulikelement 28a zur Abstützung der Misch- bzw. Fördereinrichtung 11 dient. Der Gärbehälter 1 weist jedoch zusätzlich zu den bereits aus den aus Fig. 2 bekannten Merkmalen eine zweite Fördereinrichtung 30 auf, welche einen Haupt-Gär- bzw. Faulstoffkreislauf definiert, in welchem die in dem Gär- bzw. Faulbehälter 1 befindlichen Gär- bzw. Faulstoffe durch die zweite Fördereinrichtung 30 zirkuliert werden. Die Misch- bzw. Fördereinrichtung 11, welche zusätzlich zu der zweiten Fördereinrichtung 30 angebracht ist, dient in der vorliegenden bevorzugten Ausführungsform hauptsächlich der Auflösung von unerwünschten Materialansammlungen und der Durchmischung von schlecht durchmischten Bereichen.

Die zweite Misch- bzw. Fördereinrichtung 30 weist ein Misch- bzw. Förderelement in Form eines Propellers 31, eine Antriebswelle 32 sowie eine Antriebseinheit in Form eines Motors 33 auf. Der Propeller 31 steht über die Antriebswelle 32 mit dem Motor 33 kraftschlüssig in Wirkeingriff. In der beschriebenen bevorzugten Ausführungsform erfolgt der Kraftübertrag zwischen Motor 33 und Antriebswelle 32 über ein Getriebe 34. Alternativ hierzu ist selbstverständlich jegliche andere Antriebsmethode, insbesondere ein Ketten- oder Riemenantrieb, denkbar.

Analog zur Misch- bzw. Fördereinrichtung 11 kann auch bei der zweiten Misch- bzw. Fördereinrichtung 30 an die Stelle eines Propellers 31 als Förderelement eine Schraube (beispielsweise eine schiffschraubenartig ausgebildete Schraube) oder dergleichen Vorrichtung treten. Ferner kann die zweite Fördereinrichtung 30 nicht nur, wie vorstehend beschrieben, ein so genanntes Rührwerk sein, sondern auch eine Pumpe, insbesondere eine Schneckenpumpe, Kreiselpumpe oder eine anderweitige Pumpe, welche aufgrund einer rotatorischen Bewegung eine Förderleistung erzielt bzw. welche auf einem rotatorischen Prinzip beruht.

Durch die zweite Fördereinrichtung 30 wird ein Gär- bzw. Faulstoff-Kreislauf definiert, welcher der gleichmäßigen Verteilung der Gär- bzw. Faulstoffe, welche insbesondere Abfallstoffe und nachwachsende Rohstoffe umfassen können, und einer kontinuierlichen Entgasung derselben bzw. des Gärbehälters 1 dient.

Die Vermischung erfolgt hauptsächlich mittels der zweiten Fördereinrichtung 30, welche zumindest teilweise die am Einlass 3 eintretenden Gärstoffe über eine Öffnung 35 in ein mit dieser Öffnung in Fluidverbindung stehendes Rohr 36 saugt. Die Öffnung 35 ist in einem oberen Bereich des Gärbehälters 1 bzw. des Prozessraums 2 (der Mittelpunkt der Öffnung befindet sich auf Höhe von 80 % - 90 % des Betriebsfüllstandes 6 des Gärbehälters 1) in der Nähe des Einlasses 3 angeordnet. Das Rohr 36 ist in etwa n-förmig ausgebildet, wobei der nach oben gewandte Schenkel 36 a des Rohres 36 mit dem senkrechten Abschnitt 36 b des Rohres 36 einen Winkel von größer 90° (in etwa 100° bis 110°) einschließt. Der Gärstoff, welcher durch die Öffnung 35 eingesaugt wird, wird über einen unteren Schenkel 36 c des Rohres 36, in welchem der Propeller 31 angeordnet ist, über diesen in eine im Gärbehälter angeordnete Öffnung 37, in welche der untere Schenkel 36 c des Rohres 36 hineinragt, eingespeist.

Der untere Schenkel 36 c des Rohres 36 ist in der Öffnung 37 im Behälter 1 mittels eines Kugelgelenks 38 beweglich bzw. schwenkbar gelagert. Dadurch ist der Winkel, unter dem die Gär- bzw. Faulstoffe bezüglich des Behälterbodens eingeleitet werden, variabel, sodass alle gewünschten Strömungsverhältnisse herstellbar sind. An Stelle des Kugelgelenks 38 sind auch eine entsprechende Blendenanordnung, Düsenanordnung oder jegliche andere die Strömung leitende Vorrichtung denkbar. Dadurch entsteht die gewünschte Durchmischung der Gärstoffe in einem Kreislauf, welcher von der Öffnung 35 über das Rohr 36 hin zur Öffnung 37 und dort über eine Zirkulation bzw. Strömung im Prozessraum 2 wieder zur Öffnung 35 führt. Der Gärbehälter 1 weist also in anderen Worten gesagt einen außerhalb des Prozessraums 2 angeordneten Abschnitt 39 des Gärstoff-Kreislaufs auf, welcher im Wesentlichen durch das Rohr 36 sowie Teile der zweiten Fördereinrichtung 30 (Teile der Antriebswelle 32 sowie insbesondere den Propeller 31) definiert wird. Der Mittelpunkt der Öffnung 37 befindet sich auf Höhe von 5 % - 20 % des Betriebsfüllstandes 6 des Gärbehälters 1.

Die zweite Fördereinrichtung 30 ist teilweise, sowohl in Form des Propellers 31 als auch in Form eines Abschnitts der Antriebswelle 32 in dem außerhalb des Prozessraums 2 angeordneten Kreislaufabschnitt 39, nämlich im Schenkel 36 c des Rohres 36, angeordnet. Bei alternativen bevorzugten Ausführungsformen können analog zur hier beschriebenen bevorzugten Ausführungsform beispielsweise anstelle des Propellers 31 als Misch- bzw. Förderelement eine Schraube, insbesondere eine dreiblättrige Schraube (ähnlich einer Schiffschraube) oder ein Pumpelement, beispielsweise die Schnecke einer Schneckenpumpe bzw. eine Kreiselpumpe im Kreislaufabschnitt 39 angeordnet sein. Alternativ ist es auch denkbar, dass die gesamte zweite Fördereinrichtung 30 im Kreislaufabschnitt 39 angeordnet ist. Alternativ zur Anordnung im Schenkel 36 c kann die zweite Fördereinrichtung 30 bzw. können Teile der zweiten Fördereinrichtung 30 auch in anderen Bereichen des Rohres 36 angeordnet sein (insbesondere im Schenkel 36 a oder im Abschnitt 36 b).

Im Rohr 36 sind in einem dem unteren Schenkel 36 c zugewandten Bereich des senkrechten Abschnitts 36 b des Rohres 36 sowie in einem der Öffnung 37 zugewandten Bereich des unteren Schenkels 36 c des Rohres 36 zwei Absperreinrichtungen 40, 41 in Form von Schiebern, insbesondere Plattenschiebern, Doppelplattenschiebern oder Brillenschiebern angeordnet, durch deren Schließen es ermöglicht wird, die zweite Fördereinrichtung 30, insbesondere das Förderelement 31 zu reparieren bzw. dieselbe bzw. dasselbe auszuwechseln, während der Prozessraum 2 des Gärbehälters 1 mit Gärstoffen bzw. Substrat gefüllt bleiben kann. Auch hier wären als Alternativen zu Schiebern Ventile, insbesondere Kugelventile bzw. Klappenventile mit einer oder mehreren Klappen denkbar.

Die Antriebsvorrichtung (Motor 33), welche die Antriebswelle 32 antreibt, ist über einen Flansch 42 an das Rohr 36 angeflanscht. Im Bereich des Flansches 42 ist die Antriebswelle 32 mittels geeigneter Lager, beispielsweise Rollen- bzw. Kugellager gelagert und mittels einer Dichtung fluiddicht gegen die Umgebung bzw. gegen den Motor 33 abgedichtet. Sowohl der Motor 33 als auch der außen liegende Kreislaufabschnitt sind fest im Boden verankert. Hierzu finden Verankerungselemente in Verbindung mit passenden Stützen Verwendung.

Für eine nötige Entleerung eines Volumens 43, welches im Rohr 36 durch die beiden Absperrvorrichtungen 40, 41 definiert ist, weist der untere Schenkel 36 c des Rohres 36 einen Auslass 44 auf, welcher mittels einer Absperrvorrichtung 45, die in der hier beschriebenen bevorzugten Ausführungsform in Form eines Schiebers vorliegt, verschlossen ist. Bei einem Austausch bzw. einer Reparatur der zweiten Fördereinrichtung 30 bzw. des Förderelements können die im Volumen 43 befindlichen Gärstoffe durch den Auslass 44 abgelassen werden. Der Auslass 20 ist in etwa an der am tiefsten liegenden Stelle des außen liegenden Kreislaufabschnitts 39 bzw. an der tiefstliegenden Stelle des Volumens 43 angebracht, um eine sichere Entleerung bei einer Reparatur der zweiten Fördereinrichtung 30 gewährleisten zu können. Auch die Absperrvorrichtung 45 liegt in der bevorzugten Ausführungsform in Form eines Schiebers vor, wobei hier an Stelle eines Schiebers jegliche andere Absperrvorrichtung, insbesondere in Form eines Ventils, beispielsweise eines Kugelventils oder Klappenventils, denkbar wäre. Alternativ oder zusätzlich zur Absperrvorrichtung 45 kann der Auslass 44 mit einer Verschlusskappe, welche fluiddicht schließt, versehen sein.

Es sei an dieser Stelle angemerkt, dass sämtliche Absperreinrichtungen teilweise, d.h. an den Teilen, an denen sie mit den Gärstoffen in Verbindung kommen, mit einem Korrosionsschutz in Form einer keramischen Beschichtung versehen sind. Alternativ hierzu kommt selbstverständlich auch eine Herstellung der mit den Gärstoffen in Verbindung stehenden Teile der Absperrvorrichtung aus einem korrosionsbeständigen Material, beispielsweise einer Keramik oder geeigneten Stählen, insbesondere Edelstählen oder Kunststoffen, in Frage. Auch eine Herstellung der gesamten Absperreinrichtung(en) aus korrosionsbeständigem Material bzw. eine vollständige Beschichtung der Absperreinrichtungen ist denkbar.

Es bleibt noch anzumerken, dass zur einfachen Entleerung des Volumens 43 die Absperreinrichtung 40 oberhalb bzw. stromaufwärts des Propellers 31 angeordnet ist, während die Absperreinrichtung 41 stromabwärts des Propellers 31 angeordnet ist. Die Begriffe "stromaufwärts" und "stromabwärts" beziehen sich dabei auf den normalen Betrieb der Anlage (entgegen dem Uhrzeigersinn), wobei angemerkt sei, dass die Anlage auch im umgekehrten Sinne (im Uhrzeigersinn) betrieben werden kann. Dies ist insbesondere notwendig, um eventuelle Blockaden bzw. Verstopfungen im Rohr 36 zu beseitigen. Auch für eine bessere Gasabfuhr kommt ein Betrieb entgegen der herkömmlichen Richtung in Frage.

## Patentansprüche

1. Fördersystem eines Gär- bzw. Faulbehälters (1), welches eine Fördereinrichtung (11) umfasst, welche eine Antriebsvorrichtung (14), eine Antriebswelle (13) und ein Förderelement (12) aufweist,
**dadurch gekennzeichnet,**
**dass** das Fördersystem weiterhin eine Kammer umfasst, in welche das Förderelement (12) rückziehbar bzw. einführbar ist, und welche nach einem Rückziehen bzw. Einführen des Förderelements (12) flüssigkeits- oder fluiddicht abschließbar ist.

2. Fördersystem nach Anspruch 1,
**dadurch gekennzeichnet,**
**dass** die Kammer (15) eine Kammerwand (17) aufweist, die eine Befestigungseinrichtung zur Befestigung an einer Wand (18) eines Gär- bzw. Faulbehälters (1) aufweist.

3. Fördersystem nach Anspruch 1 oder Anspruch 2,
**dadurch gekennzeichnet,**
**dass** an der Kammer (15) ein Absperrelement (16) angeordnet ist, welches alternativ geöffnet oder geschlossen werden kann; um die Kammer (15) zu öffnen oder flüssigkeitsdicht oder fluiddicht abzuschließen.

4. Fördersystem nach einem der vorhergehenden Ansprüche,
**dadurch gekennzeichnet,**
**dass** die Antriebswelle (13) mit dem daran befestigten Förderelement (12) durch das geöffnete Absperrelement (16) hindurch in die Kammer (15) einführbar und aus dieser herausführbar ist.

5. Fördersystem nach einem der vorhergehenden Ansprüche,
**dadurch gekennzeichnet,**
**dass** die Kammer wenigstens teilweise durch eine Befestigungsvorrichtung (23) welche an einem Gär- bzw. Faulbehälter (1) befestigbar ist, gebildet wird, welche ein Zwischenelement (24), welches an der Fördereinrichtung (11) angebracht ist, und ein Anschlusselement (25) aufweist, welches einerseits an dem Zwischenelement (24) angebracht und andererseits dazu ausgebildet ist, an einem/dem Gär- bzw. Faulbehälter (1) angebracht zu werden.

6. Fördersystem nach Anspruch 5,
**dadurch gekennzeichnet,**
**dass** das Zwischenelement (24) eine Zwischenplatte ist und eine Führungsbuchse (21) aufweist, welche vorzugsweise aus Kunststoff gefertigt ist, und in welcher die Antriebswelle (13) der Fördereinrichtung (11) gelagert oder geführt ist.

7. Fördersystem nach einem der Ansprüche 5 oder 6,
**dadurch gekennzeichnet,**
**dass** das Zwischenelement (24) eine Dichtung (22), insbesondere einen Simmering aufweist, welcher vorzugsweise auf einer der Fördereinrichtung (11) abgewandten Seite des Zwischenelements (24) angeordnet ist.

8. Fördersystem nach einem der Ansprüche 5 bis 7,
**dadurch gekennzeichnet,**
**dass** das Anschlusselement (25) wenigstens ein flexibles Element, insbesondere ein schlauchartiges Element aufweist.

9. Fördersystem nach einem der vorhergehenden Ansprüche,
**dadurch gekennzeichnet,**
**dass** die Fördereinrichtung (11) eine verstellbare Stütze, insbesondere eine Gewindespindel (28) oder eine Hydraulikanordnung (28a) aufweist.

10. Gär- bzw. Faulbehälter (1), insbesondere für Biogas-Anlagen bzw. biologische Kläranlagen, mit einem Prozessraum (2), einem Einlass (3) und einem Auslass (4),
**dadurch gekennzeichnet,**
**dass** der Gär- bzw. Faulbehälter (1) weiterhin ein Fördersystem nach einem der vorangehenden Ansprüche umfasst.

11. Gär- bzw. Faulbehälter (1) nach Anspruch 10,
**dadurch gekennzeichnet,**
**dass** die Kammer (15) des Fördersystems außerhalb des Prozessraums (2) angeordnet ist.

12. Gär- bzw. Faulbehälter (1) nach einem der vorhergehenden Ansprüche 10 oder 11,
**dadurch gekennzeichnet,**
**dass** der Faul- bzw. Gärbehälter (1) eine zweite Fördereinrichtung (30) aufweist, welche einen Haupt-Gär- bzw. Faulstoffkreislauf definiert, in welchem in dem Gär- bzw. Faulbehälter (1) befindliche Gär- bzw. Faulstoffe durch die zweite Fördereinrichtung (30) zirkuliert werden.

13. Gär- bzw. Faulbehälter (1) nach Anspruch 12,
**dadurch gekennzeichnet,**
**dass** der Gär- bzw. Faulbehälter (1) einen außerhalb des Prozessraums (2) angeordneten Kreislaufabschnitt (39) aufweist und die zweite Fördereinrichtung (30) wenigstens teilweise in dem außerhalb des Prozessraums (2) angeordneten Kreislaufabschnitt (39) angeordnet ist.

## Claims

1. Conveying system of a fermentation and/or digestion tank (1), which comprises a conveyor (11) which has a drive device (14), a drive shaft (13) and a conveying element (12),
**characterized in that**
the conveying system also comprises a chamber into which the conveying element (12) can be retracted and/or introduced and which, following a retraction and/or introduction of the conveying element (12), can be closed off in a liquid-tight or fluid-tight manner.

2. Conveying system according to Claim 1,
**characterized in that**
the chamber (15) has a chamber wall (17) which has a fastening means for fastening on a wall (18) of a fermentation and/or digestion tank (1).

3. Conveying system according to Claim 1 or Claim 2,
**characterized by**
a shut-off element (16) being arranged on the chamber (15), and this shut-off element can alternatively be opened or closed in order for the chamber (15) to be opened or closed off in a liquid-tight or fluid-tight manner.

4. Conveying system according to one of the preceding claims,
**characterized in that**
the drive shaft (13), with the conveying element (12) fastened thereon, can be introduced into the chamber (15), and guided out of the same, through the open shut-off element (16).

5. Conveying system according to one of the preceding claims,
**characterized in that**
the chamber is formed, at least in part, by a fastening device (23) which can be fastened on a fermentation and/or a digestion tank (1) and has an intermediate element (24), which is fitted on the conveyor (11), and a connection element (25), which, on the one hand, is fitted on the intermediate element (24) and, on the other hand, is designed to be fitted on a/the fermentation and/or digestion tank (1).

6. Conveying system according to Claim 5,
**characterized in that**
the intermediate element (24) is an intermediate plate and has a guide bushing (21) which is preferably produced from plastic and in which the drive shaft (13) of the conveyor (11) is mounted or guided.

7. Conveying system according to either of Claims 5 and 6,
**characterized in that**
the intermediate element (24) has a seal (22), in particular a shaft seal, which is preferably arranged on a side of the intermediate element (24) which is directed away from the conveyor (11).

8. Conveying system according to one of Claims 5 to 7,
**characterized in that**
the connection element (25) has at least one flexible element, in particular a hose-like element.

9. Conveying system according to one of the preceding claims,
**characterized in that**
the conveyor (11) has an adjustable support, in particular a threaded spindle (28) or a hydraulic arrangement (28a).

10. Fermentation and/or digestion tank (1), in particular for biogas plants and/or biological clarification plants, having a process space (2), an inlet (3) and an outlet (4),
**characterized in that**
the fermentation and/or digestion tank (1) also comprises a conveying system according to one of the preceding claims.

11. Fermentation and/or digestion tank (1) according to Claim 10,
**characterized in that**
the chamber (15) of the conveying system is arranged outside the process space (2).

12. Fermentation and/or digestion tank (1) according to either of preceding Claims 10 and 11,
**characterized in that**
the fermentation and/or digestion tank (1) has a second conveyor (30) which defines a main fermentation-material and/or digestion-material circuit in which fermentation and/or digestion materials located in the fermentation and/or digestion tank (1) are circulated by the second conveyor (30).

13. Fermentation and/or digestion tank (1) according to Claim 12,
**characterized in that**
the fermentation and/or digestion tank (1) has a circuit portion (39) arranged outside the process space (2), and the second conveyor (30) is arranged, at least in part, in the circuit portion (39) arranged outside the process space (2).

## Revendications

1. Système de transport d'un récipient de fermentation ou de pourriture (1), qui comprend un dispositif de transport (11) comportant un dispositif d'entraînement (14), un arbre d'entraînement (13) et un élément de transport (12), **caractérisé en ce que** le système de transport comprend en outre une chambre, dans laquelle l'élément de transport (12) peut être rétracté ou introduit, et qui peut être obturée de manière étanche au liquide ou au fluide après un retrait ou une introduction de l'élément de transport (12).

2. Système de transport selon la revendication 1, **caractérisé en ce que** la chambre (15) comprend une paroi de chambre (17), qui comporte un dispositif de fixation pour la fixation à une paroi (18) d'un récipient de fermentation ou de pourriture (1).

3. Système de transport selon la revendication 1 ou 2, **caractérisé en ce qu'**un élément de fermeture (16), qui peut être alternativement ouvert ou fermé, est disposé sur la chambre (15), afin d'ouvrir la chambre (15) ou de la fermer de manière étanche au liquide ou au fluide.

4. Système de transport selon l'une quelconque des revendications précédentes, **caractérisé en ce que** l'arbre d'entraînement (13), sur lequel l'élément de transport (12) est fixé, peut être introduit dans la chambre (15) ou rétracté hors de celle-ci à travers l'élément de fermeture (16) ouvert.

5. Système de transport selon l'une quelconque des revendications précédentes, **caractérisé en ce que** la chambre est formée au moins en partie par un dispositif de fixation (23), qui peut être fixé à un récipient de fermentation ou de pourriture (1), et qui comprend un élément intermédiaire (24), qui peut être installé sur le dispositif de transport (11), et un élément de raccordement (25), qui est d'une part installé sur l'élément intermédiaire (24) et qui est d'autre part réalisé de façon à être installé sur un/le récipient de fermentation ou de pourriture (1).

6. Système de transport selon la revendication 5, **caractérisé en ce que** l'élément intermédiaire (24) est une plaque intermédiaire et comprend une douille de guidage (21), qui est fabriquée de préférence en matière plastique et dans laquelle l'arbre d'entraînement (13) du dispositif de transport (11) est monté ou guidé.

7. Système de transport selon l'une quelconque des revendications 5 ou 6, **caractérisé en ce que** l'élément intermédiaire (24) présente un joint d'étanchéité (22), en particulier une bague d'arbre, qui est disposé de préférence sur un côté de l'élément intermédiaire (24) situé à l'opposé du dispositif de transport (11).

8. Système de transport selon l'une quelconque des revendications 5 à 7, **caractérisé en ce que** l'élément de raccordement (25) comprend au moins un élément flexible, en particulier un élément en forme de tuyau souple.

9. Système de transport selon l'une quelconque des revendications précédentes, **caractérisé en ce que** le dispositif de transport (11) comprend un appui réglable, en particulier une broche filetée (28) ou un dispositif hydraulique (28a).

10. Récipient de fermentation ou de pourriture (1), en particulier pour des installations de biogaz ou des stations d'épuration biologiques, avec une chambre de traitement (2), une entrée (3) et une sortie (4), **caractérisé en ce que** le récipient de fermentation ou de pourriture (1) comprend en outre un système de transport selon l'une quelconque des revendications précédentes.

11. Récipient de fermentation ou de pourriture (1) selon la revendication 10, **caractérisé en ce que** la chambre (15) du système de transport est disposée à l'extérieur de la chambre de traitement (2).

12. Récipient de fermentation ou de pourriture (1) selon l'une quelconque des revendications précédentes 10 ou 11, **caractérisé en ce que** le récipient de fermentation ou de pourriture (1) présente un deuxième dispositif de transport (30), qui définit un circuit principal des matières de fermentation ou de pourriture, dans lequel les matières de fermentation ou de pourriture se trouvant dans le récipient de fermentation ou de pourriture (1) sont mises en circulation au moyen du deuxième dispositif de transport (30).

13. Récipient de fermentation ou de pourriture (1) selon la revendication 12, **caractérisé en ce que** le récipient de fermentation ou de pourriture (1) comprend une partie de circuit (39) disposée à l'extérieur de la chambre de traitement (2) et le deuxième dispositif de transport (30) est installé au moins en partie dans la partie de circuit (39) disposée à l'extérieur de la chambre de traitement (2).
